# EUROPÄISCHE PATENTSCHRIFT

(11) **EP 1 345 068 B1**
(45) Veröffentlichungstag und Bekanntmachung des Hinweises auf die Patenterteilung: **25.10.2006**
(21) Anmeldenummer: 03004601.5
(22) Anmeldetag: 01.03.2003
(51) Int. Cl.: G02C 5/14, G02C 5/00, A61F 9/02, G02C 5/16

(54) **Brille, insbesondere Arbeitsschutzbrille**
Spectacles, especially working safety glasses
Lunettes de protection, notamment lunettes protectrices de travail

(30) Priorität: 14.03.2002 DE 20204052 U
(43) Veröffentlichungstag der Anmeldung: 17.09.2003
(73) Patentinhaber: Uvex Arbeitsschutz GmbH, 90766 Fürth (DE)
(72) Erfinder: Brück, Stefan, 90419 Nürnberg (DE)
(74) Vertreter: Schneck, Herbert

(56) Entgegenhaltungen:
- EP-A- 0 529 202
- WO-A-02/08819
- DE-U- 9 110 752
- DE-U- 20 103 789
- DE-U- 20 116 193
- DE-U- 20 204 052

## Beschreibung

Die Erfindung richtet sich auf eine Brille, insbesondere eine Arbeitsschutzbrille, mit gepolsterten Brillen-Bügeln.

Eine derartige Brille ist aus EP 0 529 202 B1 bekannt. Bei dieser bekannten Ausgestaltung ist vorgesehen, dass am Bügel-Ende eine ösenartige Konfiguration aus relativ hartem Kunststoff ausgebildet ist, die von einer sich auf den Kopf des Benutzers zu vorwölbenden Blase überspannt wird. Hierdurch wird unmittelbar am Bügel-Ende ein guter Sitz und ein angenehmes Tragegefühl erreicht.

Hiervon ausgehend liegt der Erfindung die Aufgabe zugrunde, eine Arbeitsschutzbrille mit Bügeln der eingangs genannten Art so weiterzubilden, dass auch der Auflagebereich längs der Unterkante, also am Ohr des Benutzers, so ausgestaltet ist, dass ein möglichst großer Tragekomfort bei festem Sitz erzielt wird.

Diese Aufgabe wird erfindungsgemäß dadurch gelöst, dass im Ohrauflagebereich jedes Bügels die Oberseite des Bügels aus einem Steg aus einem relativ hartem Kunststoff besteht, wobei unterhalb dieses Steges ein Bereich aus relativ weichem Kunststoff mit einer im wesentlichen durchgehenden Unterkante ausgebildet ist, und wobei zwischen der Unterkante bzw. dem dort verlaufenden Steg und dem oberen Steg in dem weichen Kunststoff wenigstens eine blasenartige Vorwölbung in Richtung auf den Kopf des Benutzers zu ausgebildet ist.

Insbesondere kann vorgesehen sein, dass eine Mehrzahl von blasenartigen Vorwölbungen ausgebildet ist, die durch annähernd vertikale Stege aus weichem Kunststoff voneinander getrennt sind, die sich zwischen dem Steg aus hartem Kunststoff und der Unterkante bzw. dem unteren Steg erstrecken.

Durch die erfindungsgemäße Ausgestaltung wird bei ausreichender Grundstabilität ein sehr weicher Ohrauflagebereich geschaffen, der sich im gewissen Umfang von der Form her anpassen kann und neben einem hohen Tragekomfort auch eine zuverlässige Positionierung gewährleistet.

Das Bügel-Ende kann in an sich bekannter Weise durch eine ösenartige Verbreiterung des harten Kunststoffmaterials gebildet sein, über welche sich blasenartig vorspringend weiches Kunststoffmaterial erstreckt.

Günstigerweise ist jeder Brillen-Bügel in einer Spritzgieß-Form durch je einen Schuss aus hartem Material und weichem Material hergestellt.

Nachfolgend wird die Erfindung anhand eines bevorzugten Ausführungsbeispiels in Verbindung mit der Zeichnung näher erläutert. Dabei zeigen:

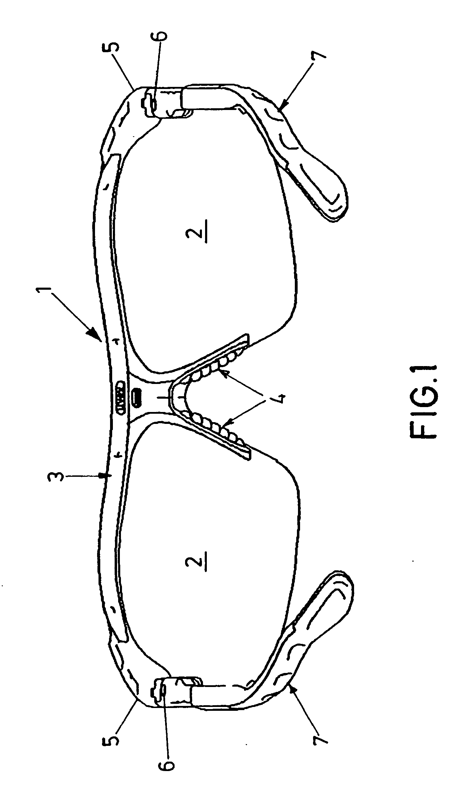

Fig. 1 eine Ansicht einer erfindungsgemäßen Brille von hinten bzw. aus der Sicht des Trägers,

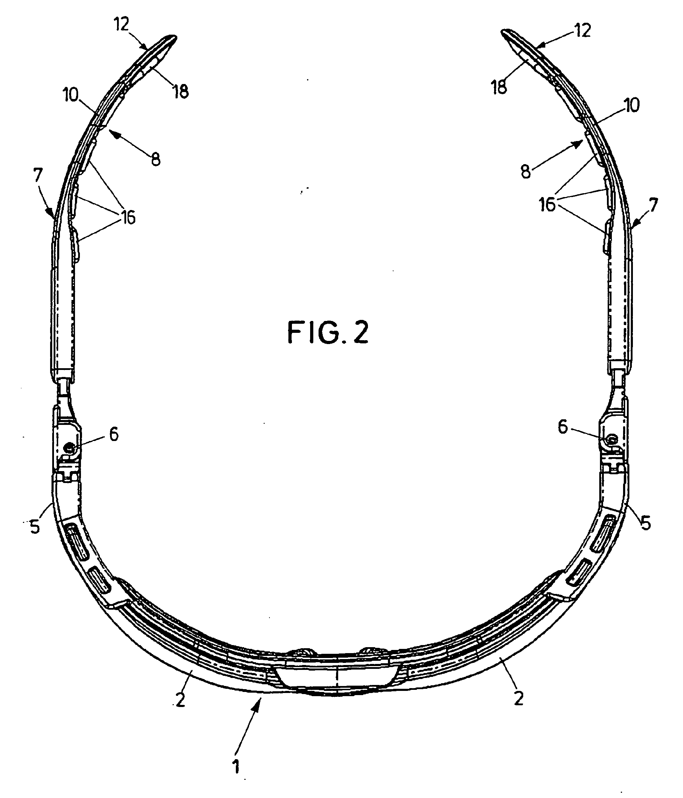

Fig. 2 eine Draufsicht einer erfindungsgemäßen Brille,

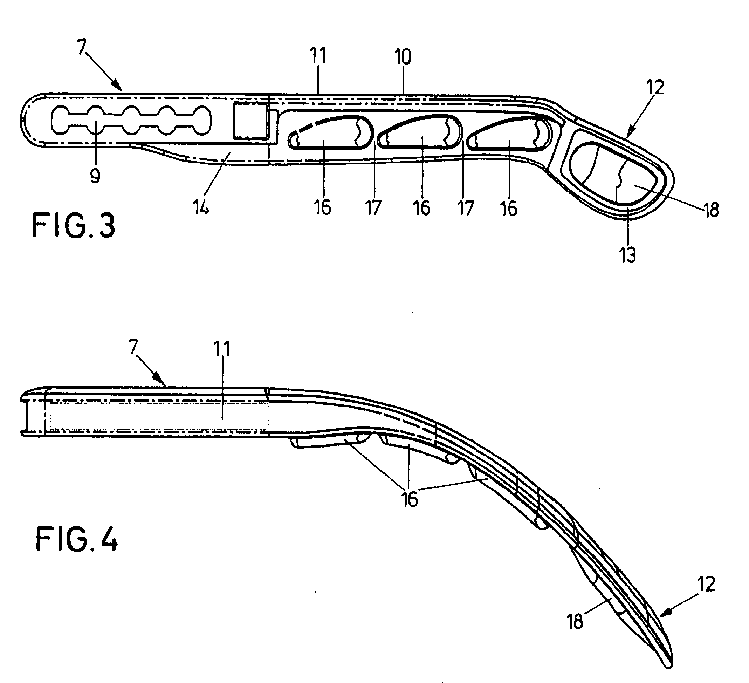

Fig. 3 eine vergrößerte Seitenansicht eines Bügels von innen und
Fig. 4 eine vergrößerte Draufsicht eines Bügels.

In der Zeichnung ist eine Arbeitsschutzbrille 1 dargestellt, die zwei Scheiben 2 umfasst, welche an einem oberen Rahmenteil 3 mit Nasenauflagebereich 4 angeordnet sind, wobei an den äußeren Enden 5 des oberen Rahmenteils 3 über Scharniere 6 in an sich bekannter Weise Bügel 7 angelenkt sind. Die Bügel 7 sind im Kopfanlagebereich 8 nach innen gebogen.

Der innere Endbereich jedes Bügels 7 ist in an sich bekannter Weise über eine Rastausnehmung 9 zur Erzielung einer Längenverstellbarkeit mit dem äußeren Ende 5 des oberen Rahmenteils 3 verbunden. Der Abschnitt 7 des Bügels besteht aus einem relativ harten Kunststoff, der sich in Form eines Steges 10 die Oberkante 11 des Bügels 7 ausbildend nach hinten bis zu dem Bügel-Ende 12 erstreckt, wo der harte Kunststoff eine verbreiterte ösenartige Konfiguration 13 ausbildet.

An den harten Kunststoff bzw. den Steg 10 ist ein weicher Kunststoff angespritzt, der sich über den unteren Endbereich 14 des inneren Endabschnitts des Bügels 7 erstreckt und von dort ausgehend nach Art eines Steges 15 die auf dem Ohr aufliegende Unterkante des Bügels 7 ausbildet.

Zwischen dem unteren Steg 15 aus weichem Kunststoff und dem oberen Steg 10 aus hartem Kunststoff erstreckt sich flächig der weiche Kunststoff, wobei dieser eine Mehrzahl blasenartiger Vorwölbungen 16 aufweist, die durch vertikale stegartige Abschnitte 17 voneinander getrennt sind.

Der weiche Kunststoff erstreckt sich auch über die ösenartige Verdickung 13 am Bügel-Ende 12 und ist auch dort als blasenartige Vorwölbung 18 ausgebildet.

## Patentansprüche

1. Brille, insbesondere Arbeitsschutzbrille, mit gepolsterten Brillen-Bügeln, wobei die Polsterung durch wenigstens eine blasenartige Vorwölbung (16, 18) in Richtung auf den Kopf des Trägers zu ausgebildet ist, wobei im Ohrauflagebereich jedes Bügels (7) die Oberseite (11) des Bügels (7) aus einem stegartigen Bereich (10) aus einem relativ harten Kunststoff besteht, **dadurch gekennzeichnet, dass** unterhalb des stegartigen Bereiches (10) ein Bereich (15) aus relativ weichem Kunststoff mit einer stegartigen, durchgehenden Unterkante aus relativ weichem Kunststoff ausgebildet ist, und die blasenartige Vorwölbung (16, 18) zwischen der relativ weichen stegartigen Unterkante und dem oberen relativ harten stegartigen Bereich aus weichem Kunststoff ausgebildet ist.

2. Brille nach Anspruch 1, **dadurch gekennzeichnet, dass** eine Mehrzahl von blasenartigen Vorwölbungen (16, 18) vorgesehen ist, die durch annähernd vertikale Stege (10, 15) aus weichem Kunststoff voneinander getrennt sind, die sich zwischen dem Steg (10) aus hartem Kunststoff und der Unterkante (15) erstrecken.

3. Brille nach Anspruch 1, **dadurch gekennzeichnet, dass** das Bügel-Ende (12) durch eine ösenartige Verbreiterung (13) des harten Kunststoffmaterials gebildet ist, über welche sich blasenartig vorspringend weiches Kunststoffmaterial erstreckt.

4. Verfahren zur Herstellung einer Brille nach einem der Ansprüche 1 bis 3, **dadurch gekennzeichnet, dass** der Brillenbügel (7) in einer Form durch je einen Schuss aus hartem Kunststoff-Material und weichem Kunststoff-Material hergestellt ist.

## Claims

1. A pair of goggles, particularly a pair of safety goggles, having padded ear pieces, wherein the padding is formed by at least one bubble-like convexity (16, 18) in a direction towards the head of the user, wherein in the ear contact area of each ear piece (7) the upper side (11) of the ear piece (7) consists of a strip-like area (10) made of a relatively hard plastic material, **characterized in that** below the strip-like area (10) an area (15) made of relatively soft plastic material and having a strip-like continuous lower edge made of relatively soft plastic material is formed, and the bubble-like convexity (16, 18) is composed of a soft plastic material between the relatively soft strip-like lower edge and the upper, relatively hard strip-like area.

2. A pair of goggles according to claim 1, **characterized in that** a plurality of bubble-like convexities (16, 18) are provided which are separated from one another by approximately vertical strips (10, 15) made of soft plastic material, which strips extend between the strip (10) made of hard plastic material and the lower edge (15).

3. A pair of goggles according to claim 1, **characterized in that** the end (12) of the ear piece is formed by an eye-like enlargement (13) of the hard plastic material across which bubble-like convex soft plastic material extends.

4. Method for producing a pair of goggles according to one of claims 1 to 3, **characterized in that** the ear piece (7) is made in a mould by one injection of hard plastic material and one of soft plastic material.

## Revendications

1. Lunettes, en particuliers lunettes de protection au travail, à branches matelassées, le matelassage étant réalisé par au moins un bombement (16, 18) en forme de bulle du côté de la tête du porteur, la partie supérieure (11) de la branche (7) étant dans la zone d'appui sur l'oreille de chaque branche (7) réalisée dans une matière plastique relativement dure depuis une partie en forme de méplat (10), **caractérisées en ce qu'**en dessous de la partie en forme de méplat (10), une partie (15) en matière plastique relativement molle est formée avec un bord inférieur continu en forme de méplat en matière plastique relativement molle, et **en ce que** le bombement (16, 18) en forme de bulle entre le bord inférieur relativement mou et la partie supérieure relativement dure est formé en matière plastique molle.

2. Lunettes selon la revendication 1, **caractérisées en ce qu'**il est prévu une pluralité de bombements (16, 18) en forme de bulle séparés entre eux par des barrettes sensiblement verticales (10, 15) en matière plastique molle s'étendant entre le méplat (10) en matière plastique dure et le bord inférieur (15).

3. Lunettes selon la revendication 1, **caractérisées en ce que** l'extrémité de branche (12) est formée par un élargissement (13) en forme d'oeil de la matière plastique dure, sur lequel s'étend une matière plastique molle saillant en forme de bulle.

4. Procédé de fabrication de lunettes selon l'une des revendications 1 à 3, **caractérisé en ce que** la branche (7) est fabriquée dans un moule par une injection respective de matière plastique dure et de matière plastique molle.
